# EUROPÄISCHE PATENTSCHRIFT

(11) **EP 3 558 196 B1**
(45) Veröffentlichungstag und Bekanntmachung des Hinweises auf die Patenterteilung: **26.10.2022**
(21) Anmeldenummer: 17829622.4
(22) Anmeldetag: 15.12.2017
(51) Int. Cl.: A61F 13/15

(54) **VERFAHREN ZUM HERSTELLEN VON INKONTINENZWEGWERFWINDELN**
METHOD FOR PRODUCING DISPOSABLE INCONTINENCE PADS
PROCÉDÉ DE FABRICATION DE COUCHES JETABLES D'INCONTINENCE

(30) Priorität: 23.12.2016 DE 102016125560
(43) Veröffentlichungstag der Anmeldung: 30.10.2019
(73) Patentinhaber: Paul Hartmann AG, 89522 Heidenheim (DE)
(72) Erfinder: KNECHT, Theresia, 73433 Aalen (DE)
(86) Internationale Anmeldenummer: PCT/EP2017/082950
(87) Internationale Veröffentlichungsnummer: WO 2018/114635

(56) Entgegenhaltungen:
- EP-A1- 1 941 853
- WO-A1-2011/101772
- DE-A1-102008 046 358
- DE-A1-102014 110 941

## Beschreibung

Die Erfindung betrifft ein Verfahren zum Herstellen einer Vielzahl von einen Windelhauptteil und daran angefügte Windelseitenteile aufweisenden Inkontinenzwegwerfwindeln des offenen Typs für Erwachsene. Derartige Inkontinenzwegwerfwindeln sind bekannt und weisen einen Hauptteil, bestehend aus einem Vorderbereich, einem Rückenbereich und einem in Längsrichtung dazwischen liegenden zwischen den Beinen eines Benutzers zu liegen kommenden Schrittbereich auf, wobei der Hauptteil meist bereits einen Absorptionskern umfasst, und mit an den Rückenbereich beidseits angefügten, voneinander separaten Windelseitenteilen, welche sich in Querrichtung über seitliche Längsränder des Hauptteils hinaus erstrecken und den Vorderbereich und den Rückenbereich im angelegten Zustand des Artikels miteinander verbinden.

Die Seitenteile werden vorzugsweise im Cut&Place-Verfahren (Slip-Cut) direkt, beidseitig an den Windelhauptteil, also das Chassis des Hygieneartikels angefügt. Diese Fertigungstechnologie erlaubt es, die Seitenteile aus einem anderen Material zu fertigen als den Windelhauptteil des Hygieneartikels. Beispielsweise können die Windelseitenteile luftdurchlässig ausgeführt werden, wohingegen der Windelhauptteil im Wesentlichen feuchtigkeitsundurchlässig ausgebildet werden kann.

Die aus der Fertigungssicht effizienteste und einfachste sowie kostengünstigste Form der Windelseitenteile ist die rechteckige Form. Sie erlaubt bei der Herstellung den Transport der die Windelseitenteile bildenden Materialien in Form einer endlosen Flachmaterialbahn, von der dann die Windelseitenteile quer zur Maschinenrichtung abgetrennt werden. Ein Schnittabfall ist hier praktisch nicht gegeben.

Es hat sich jedoch gezeigt, dass insbesondere bei der Ausbildung der Windelseitenteile in der ansonsten vorteilhaften Rechteckform beim Anlegen und Tragen des Hygieneartikels mitunter das Problem besteht, dass die angefügten Windelseitenteile im Bereich der seitlichen Längsränder des Windelhauptteils einreißen können. Die Anwender sind nämlich geneigt, beim Anlegen des Hygieneartikels einen zur Quer- und Längsrichtung des Hygieneartikels schrägen Zug auf die im Falle von Inkontinenzartikeln für Erwachsene extrem weit in Quer-und Längsrichtung ausladenden Windelseitenteile auszuüben. Es kann solchenfalls vorkommen, dass Windelseitenteile entlang der seitlichen Längsränder des Windelhauptteils einreißen, wobei der Riss ausgehend von dem dem Schrittbereich zugewandten Querrand des Windelseitenteils ausgeht. Die DE 102010026643 schlägt daher vor, die hinteren Windelseitenteile mit einer Konturierung zu versehen. Die DE102010049171 zeigt darüber hinaus bereits ein Verfahren zur Herstellung einer Inkontinenzwegwerfwindel mit konturierten Windelseitenteilen. So zeigt die DE 102010049171 auch bereits die weitestgehend materialverlustfreie Erzeugung von Windelseitenteilen aus einer endlosen Materialbahn von der schräg zur Maschinenrichtung der Materialbahn doppelnutzige Materialbahnabschnitte in alternierender Abfolge abgetrennt werden. Auch WO2008141834 und DE102008046358A1 offenbaren Verfahren zur Herstellung von Inkontinenzwegwerfwindeln der eingangs beschriebenen Art. Es hat sich jedoch gezeigt, dass das präzise Führen der Materialbahn in schnell laufenden Windelherstellungsmaschinen mit Schwierigkeiten versehen ist. Beim Schneiden und Abtrennen der Materialbahnabschnitte von der Materialbahn muss die Materialbahn unter Zug gehalten werden, was angesichts der erforderlichen Breite der Materialbahn und der oft geringen Flächengewichte der verwendeten Materialien problematisch ist. Die WO2016016384A1 schlägt daher ein Verfahren vor, bei dem doppelnutzige Materialbahnabschnitte von einer bereits vorgefalteten Materialbahn abgetrennt werden, wobei die Materialbahn vor der Faltung eingeschnitten wird, wobei sich die Schnitte aber nicht bis zu den Materialbahnrändern erstrecken.

Der vorliegenden Erfindung liegt die Aufgabe zu Grunde, ein verbessertes Verfahren zum Herstellen von derartigen oder ähnlichen Inkontinenzwegwerfwindeln zu schaffen, das kostengünstig und prozesstechnisch vorteilhaft realisierbar ist.

Diese Aufgabe wird erfindungsgemäß gelöst durch ein Verfahren gemäß Anspruch 1.

Vorteilhafterweise erstrecken sich die ersten Trennlinien über 50-95 % einer Breite der Materialbahn, insbesondere über 60-90 % der Materialbahn, insbesondere über 65-85% der Materialbahn.

Durch das Einfalten der Bahn wird die Breite der Bahn reduziert. Auch das erleichtert die nachfolgenden Verfahrensschritte. Die in der Maschinenrichtung alternierende Anordnung der von der Bahn abzutrennenden einnutzigen Windelseitenteilabschnitte erlaubt eine weitestgehend materialverlustfreie Bereitstellung der Windelseitenteile. Die alternierende Anordnung der von der Bahn abzutrennenden einnutzigen Windelseitenteilabschnitte bedeutet im Zusammenhang mit der vorliegenden Erfindung, dass ein jeweiliger einnutziger Windelseitenteilabschnitt in der Ebene um 180° gedreht angeordnet ist gegenüber einem unmittelbar in der Maschinenrichtung nachfolgenden einnutzigen Windelseitenteilabschnitt. Somit bildet der erste Materialbahnrand einen inneren Rand eines ersten einnutzigen Windelseitenteilabschnittes und der zweite Materialbahnrand einen äußeren Rand des ersten einnutzigen Windelseitenteilabschnittes und der zweite Materialbahnrand einen inneren Rand eines zweiten einnutzigen Windelseitenteilabschnittes und der erste Materialbahnrand einen äußeren Rand des zweiten einnutzigen Windelseitenteilabschnittes, wobei der zweite einnutzige Windelseitenteilabschnitt in der Maschinenrichtung unmittelbar auf den ersten einnutzigen Windelseitenteilabschnitt folgt. Hierbei ist ein innerer Rand eines Windelseitenteilabschnitts derjenige Rand, welcher im weiteren Verlauf der Herstellung der Inkontinenzwegwerfwindel an den Seitenrand einer Hauptteilbahn angefügt wird. Besonders bevorzugt bildet ein erster einnutziger Windelseitenteilabschnitt ein späteres linkes Windelseitenteil und ein sich unmittelbar an den ersten einnutzigen Windelseitenteilabschnitt anschließender zweiter einnutziger Windelseitenteilabschnitt ein rechtes Windelseitenteil.

Die ersten Trennlinien können vorteilhaft lediglich durch sich durch die Materialbahn bereichsweise in z-Richtung hindurcherstreckende Schnitte ausgeführt sein. Die Trennlinien können auch als diskontinuierlich, gleichsam als Schwächungslinie ausgeführt sein. Alternativ bilden die Trennlinien hingegen Materialaussparungen, derart, dass jeweils eine langgestreckte Öffnung insbesondere in ovaler Form oder einem Oval ähnlicher Form ausgebildet wird. Solchenfalls ist eine jeweilige Öffnung in der Ebene der Materialbahn vollumfänglich von Material der Materialbahn umschlossen.

Vorteilhaft sind in der Maschinenrichtung aufeinanderfolgende erste Trennlinien der Materialbahn jeweils symmetrisch in Bezug auf eine Spiegelachse quer zur Maschinenrichtung ausgerichtet. Vorzugsweise entspricht diese Spiegelachse der Lage einer gedachten Linie verlaufend in der Querrichtung, welche ein späteres Windelseitenteil in der Längsrichtung halbiert.

Die Breite der noch ungefalteten Materialbahn entspricht im Wesentlichen der Quererstreckung der späteren Windelseitenteile im entfalteten Zustand und beträgt vorzugsweise 10-50 cm, insbesondere 12-45 cm, weiter insbesondere 15-35 cm.

Die Breite der gefalteten Materialbahn beträgt vorzugsweis 5-30 cm, insbesondere 6-25 cm. Es hat sich als vorteilhaft erwiesen, dass die Faltanordnung zumindest eine Z-Falte umfasst. Nach weiteren vorteilhaften Varianten wird die Materialbahn um wenigstens drei, insbesondere um wenigstens vier in Maschinenrichtung verlaufenden Falzlinien gefaltet. Vorzugsweise kreuzen wenigstens zwei, insbesondere wenigstens drei, weiter insbesondere wenigstens vier der Falzlinien die ersten Trennlinien.

Es hat sich als weiter vorteilhaft erwiesen, wenn die in Maschinenrichtung verlaufenden Falzlinien beidseits und insbesondere symmetrisch zu einer Mittellängsachse der Materialbahn angeordnet sind. Insbesondere vorteilhaft ist, wenn die Faltanordnung in Form einer Omega-Faltung ausgebildet ist.

Nach einer alternativen Verfahrensführung können die Falzlinien asymmetrisch zu der Mittellängsachse der Materialbahn angeordnet sein. Insbesondere kann die Faltanordnung zwei Z-Falten umfassen oder daraus bestehen, wobei die eine Z-Falte vorzugsweise in die andere Z-Falte eingeschoben ist, also Teilabschnitte jeweiliger Z-Falten aufeinander zu liegen kommen.

Insbesondere erstreckt sich die Faltanordnung in der Breite der Materialbahn nicht über den ersten und zweiten Materialbahnrand der Materialbahn hinaus. Mithin bilden der erste und zweite Materialbahnrand der noch ungefalteten Materialbahn auch den ersten und zweiten Materialbahnrand der gefalteten Materialbahn.

Es ist weiter vorteilhaft wenn bei der Vereinzelung der einnutzigen Windelseitenteilabschnitte die Abtrennung von der Materialbahn durch Ausbildung zweiter und dritter Trennlinien ausgehend von einem jeweiligen Materialbahnrand der Materialbahn bis zu der ersten Trennlinie erfolgt. Die erste Trennlinie bildet mithin zumindest bereichsweise die Kontur der späteren Beinöffnungsbereiche der Windelseitenteile. Die Abtrennung kann durch an sich beliebige Trennverfahren insbesondere Schneiden, Stanzen oder Abreißen erfolgen. Auch könnten nach einer weiteren Variante Schwächungslinien wie Perforationen oder Ähnliches eingebracht werden, die - vorzugsweise in einer nachfolgenden Verfahrensstufe - die Abtrennung der einnutzigen Windelseitenteilabschnitte insbesondere durch Ausübung von Zug- oder Druckkräften ermöglichen.

Die Windelseitenteilabschnitte sind erfindungsgemäß einnutzig ausgeführt. Das bedeutet, dass ein einziger einnutziger Windelseitenteilabschnitt ein einziges Windelseitenteil bildet.

Nach einer vorteilhaften Weiterbildung der Erfindung können die einnutzigen Windelseitenteilabschnitte nach deren Vereinzelung um wenigstens je eine weitere in Maschinenrichtung verlaufende Falzlinie gefaltet werden.

Im weiteren Verlauf der Herstellung der Windel kann es vorteilhaft sein, die einnutzigen Windelseitenteilabschnitte einer in der Windelmaschinenrichtung geförderten Hauptteilbahn zuzuführen und die Windelseitenteilabschnitte zur Bildung von späteren Windelseitenteilen beidseitig an der Hauptteilbahn anzufügen. Dies kann vorteilhaft mit dem Fachmann an sich bekannten Vorrichtungen, wie so genannten Cut&Place (Slip-Cut) - Applikatoren erfolgen.

Insbesondere werden die einnutzigen Windelseitenteilabschnitte ausschließlich an einen Rückbereich eines Hauptteils angefügt. Ein jeweiliger Vorder- oder Bauchbereich eines Hauptteils bleibt also windelseitenteilfrei und wird durch jeweilige seitliche Längsränder des Hauptteils begrenzt. Es werden mithin durch das Verfahren insbesondere sogenannte T-Form Windeln bereitgestellt.

Die Hauptteilbahn kann vorteilhaft einen Verbund aus einem Backsheet, einem Topsheet und einen dazwischen angeordneten Saugkörper aufweisen. Die Hauptteilbahn kann nach einer Variante hingegen aus lediglich einer oder zweier dieser Komponenten bestehen. Des Weiteren kann die Hauptteilbahn vorzugsweise eine so genannte Cuff-Komponente aufweisen oder daraus bestehen. Als Cuff-Komponenten werden üblicherweise in Gebrauch flüssigkeitsabweisende oder hydrophobe Flachmaterialien, insbesondere auf Vliesstoffbasis bezeichnet, welche eine sich im Wesentlichen über die gesamte Länge zumindest des Schrittbereiches der Windel erstreckende seitliche Auslaufschutzbarriere bilden und vorzugsweise Teil der körperzugewandten Oberseite der Windel sind. Solchenfalls würde die Cuff-Komponente mit daran angefügten spätere Windelseitenteile bildenden einnutzigen Windelseitenteilabschnitten in einer nachfolgenden Verfahrensstufe mit weiteren, den Windelhauptteil bildenden Komponenten verbunden werden.

Vorteilhafterweise wird die Materialbahn in der Maschinenrichtung mit einer ersten Geschwindigkeit v1 gefördert und die Hauptteilbahn in der Windelmaschinenrichtung mit einer zweiten Geschwindigkeit v2 gefördert, wobei gilt v1 kleiner v2. Solchenfalls werden die einnutzigen Windelseitenteilabschnitte vorteilhafterweise nach der Abtrennung von der Materialbahn im Zuge der Applikation auf die Windelhauptteilbahn beschleunigt, insbesondere auf eine Geschwindigkeit v3, die im Wesentlichen v2 entspricht.

Vorteilhaft erfolgt nach dem Anfügen der einnutzigen Windelseitenteilabschnitte an die Hauptteilbahn an einer Windelvereinzelungsstation die Vereinzelung der zuvor noch endlosen Hauptteilbahn mit daran angefügten einnutzigen Windelseitenteilabschnitten quer zur Maschinenrichtung. Es ist vorteilhaft, dass vor dem Abtrennen der einnutzigen Windelseitenteilabschnitte von der Materialbahn Verschlussmittel auf die Materialbahn aufgebracht und dort festgelegt werden. Die Verschlussmittel werden häufig auch als Tapes bezeichnet, welche die klebenden oder mechanischen Verschlusselemente aufweisen.

Ein bevorzugtes Verfahren ist dadurch gekennzeichnet, dass bei der Vereinzelung der einnutzigen Windelseitenteilabschnitte quer oder schräg zu der Maschinenrichtung die einnutzigen Windelseitenteilabschnitte direkt von der Materialbahn abgetrennt werden.

Ein weiteres bevorzugtes Verfahren ist dadurch gekennzeichnet, dass vor der Vereinzelung der einnutzigen Windelseitenteilabschnitte quer oder schräg zu der Maschinenrichtung zunächst je ein endloser Materialstreifen an dem ersten und dem zweiten Materialbahnrand der Materialbahn angefügt werden. Insbesondere wird die Materialbahn anschließend in eine erste endlose Materialstreifenbahn und eine zweiten endlose Materialstreifenbahn getrennt, derart, dass jede der Materialstreifenbahnen eine in der Maschinenrichtung getaktete Folge von einnutzigen Windelseitenteilabschnitten aufweist

Das die Materialbahn bildende Material kann quer zur Maschinenrichtung elastisch oder unelastisch ausgebildet sein.

Vorzugsweise weist die Materialbahn quer zu der Maschinenrichtung mindestens drei Abschnitte auf, umfassend einen ersten Abschnitt, ein an den ersten Abschnitt angrenzenden zweiten Abschnitt und ein an den zweiten Abschnitt angrenzenden dritten Abschnitt. Weiter vorzugsweise ist vorgesehen, dass der zweite Abschnitt ein quer zu der Maschinenrichtung elastisches Material umfasst oder daraus besteht. Insbesondere ist vorgesehen, dass der erste und dritte Abschnitt ein quer zu der Maschinenrichtung im Wesentlichen unelastisches Material umfasst oder daraus besteht.

Es hat sich als vorteilhaft erwiesen, die Windeln derart zu fertigen, dass bei in der Maschinenrichtung aufeinanderfolgend geförderten Windeln der Rückenbereich der einen Windel an den Vorderbereich der anderen Windel anschließt.

Es hat sich weiter als vorteilhaft erwiesen, die vorderen und/oder hinteren Windelseitenteile aus einem Vliesstoffmaterial zu bilden, welches zumindest eine Rezepturkomponente auf Basis eines thermoplastischen Polymers enthält. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht. Besonders bevorzugt sind Spinnvliese, da diese eine hohe Festigkeit in Längs- und Querrichtung aufweisen und somit den auf sie durch das Eingreifen von gegebenenfalls vorhandenen mechanischen Verschlusshilfen einwirkenden Scherkräften besonders gut standhalten können.

Nach dem vorstehend beschriebenem Verfahren ist eine Inkontinenzwegwerfwindel herstellbar mit einem Hauptteil, wobei der Hauptteil einen Rückenbereich umfasst, und mit beidseits an den Rückenbereich angefügten Windelseitenteilen, wohingegen an den Vorderbereich keine Windelseitenteile angefügt sind und wobei die Windelseitenteile einen oberen Rand und einen unteren Rand aufweisen, wobei der obere Rand und der untere Rand schräg zur Längsrichtung der Inkontinenzwegwerfwindel oder konturiert verlaufen und der untere Rand einen schräg zur Längsrichtung der Inkontinenzwegwerfwindel verlaufenden oder konturierten Beinöffnungsbereich begrenzt.

Der Hauptteil weist einen hinteren Querrand auf, wobei ein Abstand d der Windelseitenteile von dem hinteren Querrand in der Längsrichtung vorzugsweise wenigstens 1 mm, insbesondere wenigstens 5 mm, insbesondere wenigstens 10 mm, insbesondere wenigstens 15 mm, insbesondere höchstens 50 mm beträgt. Hierdurch ist gewährleistet, dass die beim Anlegen über die Verschlussmittel ausgeübten und dabei in den Rückbereich des Hauptteils eingeleiteten Querzugkräfte auf einen größeren Abschnitt des Hauptteils "verteilt" werden, was der Ausreißgefahr der Windelseitenteile entgegenwirkt.

Eine Erstreckung Q der eben ausgebreiteten Windelseitenteile in der Querrichtung der Inkontinenzwegwerfwindel über den hinteren seitlichen Längsrand des Hauptteils hinaus beträgt vorzugsweise 150 bis 350 mm, insbesondere 170 bis 300 mm.

Eine Erstreckung B der Windelseitenteile in der Längsrichtung beträgt im Bereich der Anfügung an den Hauptteil 100 bis 200 mm, insbesondere 120 bis 170 mm.

Es hat sich bei der erfindungsgemäßen Inkontinenzwegwerfwindel als besonders vorteilhaft erwiesen, wenn die Erstreckung Q der Windelseitenteile in der Querrichtung über den jeweiligen hinteren seitlichen Längsrand hinaus und eine maximale Erstreckung B der Windelseitenteile in der Längsrichtung derart bemessen ist, dass das Verhältnis der Erstreckungen Q/B zueinander 1,0 < Q/B < 2,0 beträgt.

Für die eingefaltete Konfiguration der Windelseitenteile gilt vorzugsweise, dass eine Erstreckung A der auf sich selbst gefalteten Windelseitenteile in der Querrichtung über den jeweiligen hinteren seitlichen Längsrand hinaus und eine Erstreckung B der auf sich selbst gefalteten hinteren Seitenabschnitte in der Längsrichtung derart bemessen ist, dass das Verhältnis der Erstreckungen A/B zueinander 0,25 < A/B < 1,50 insbesondere 0,5 < A/B < 1,0 beträgt.

Weiter erweist sich als vorteilhaft, wenn eine in der Querrichtung erstreckte und das jeweilige Verschlussmittel auf der schrittzugewandten Seite tangierende gerade Linie den Saugkörper schneidet. Dies kann vorzugsweise dann realisiert werden, wenn die beiden hinteren Seitenabschnitte wie vorstehend erwähnt einen Abstand in der Längsrichtung zum hinteren Querrand des Hauptteils aufweisen. Insbesondere ist vorgesehen, dass eine in der Querrichtung erstreckte und die Seitenabschnitte in der Längsrichtung, im Bereich der Anfügung an den Hauptteil halbierende gerade Linie den Saugkörper schneidet. Es stabilisiert die Anlage des Saugkörpers und unterstützt einen korrekten Sitz der Windel.

Weiter erweist sich als vorteilhaft, wenn eine in der Querrichtung erstreckte und das jeweilige Verschlussmittel auf der schrittzugewandten Seite gerade tangierende Linie den Saugkörper schneidet. Dies kann vorzugsweise dann realisiert werden, wenn die beiden Windelseitenteile wie vorstehend erwähnt einen Abstand in der Längsrichtung zum hinteren Querrand des Hauptteils aufweisen. Insbesondere ist vorgesehen, dass eine in der Querrichtung erstreckte und die Windelseitenteile in der Längsrichtung, im Bereich der Anfügung an den Hauptteil halbierende gerade Linie den Saugkörper schneidet. Es stabilisiert die Anlage des Saugkörpers und unterstützt einen korrekten Sitz der Windel.

Es erweist sich weiter als vorteilhaft, wenn jedes Windelseitenteil genau ein Verschlussmittel aufweist. Es handelt sich bei den Verschlussmitteln typischerweise um eine Lasche aus einem ein- oder mehrschichtigen Flachmaterial, welches ausgehend von einer in der Regel um einen distalen Längsrand des betrachteten Windelseitenteils nach innen auf das Windelseitenteil eingefalteten Konfiguration in eine nach außen ausgefaltete Betriebsstellung entfaltbar ist. In an sich bekannter und daher nicht näher zu beschreibender Weise ist ein jeweiliges Verschlussmittel mit klebenden und/oder mechanisch haftenden Bereichen, Schichten oder Elementen ausgestattet, wie zum Beispiel Haken/Schlaufen-Materialien. Sofern das Windelseitenteil genau ein Verschlussmittel aufweist, so erweist es sich als vorteilhaft, wenn dieses Verschlussmittel in der Längsrichtung ungefähr mittig in einem distalen Bereich des Windelseitenteils vorgesehen ist. Weiter erweist es sich als vorteilhaft, wenn das betreffende Verschlussmittel eine Erstreckung in der Längsrichtung zwischen 25 % und 75 % der Erstreckung B des Windelseitenteils in der Längsrichtung beträgt. Ferner sind die jeweiligen Verschlussmittel vorzugsweise im ein- und ausgefalteten Zustand rechteckförmig ausgebildet. Sie sind in der herstellerseitigen nicht aktiven Konfiguration vorzugsweise nach innen auf sich selbst eingefaltet oder auf das Windelseitenteil zurückgefaltet.

Hinsichtlich der Abmessungen des Hauptteils der Inkontinenzwegwerfwindel hat es sich als vorteilhaft erwiesen, wenn die Erstreckung des Hauptteils in der Querrichtung im Rückenbereich und/oder im Vorderbereich 250 bis 550 mm, insbesondere 300 bis 520 mm beträgt. Vorzugsweise weisen der Vorderbereich und der Rückenbereiche des Hauptteils dieselbe Quererstreckung (gemessen in mm) auf.

Die Erstreckung des Hauptteils in der Längsrichtung beträgt vorzugsweise 700 bis 1200 mm, insbesondere 800 bis 1100 mm.

Der Hauptteil kann im Schrittbereich mit einer Verengung in Querrichtung, mithin mit einer Beinöffnungskontur versehen sein. In einer alternativen Ausführungsform ist der Hauptteil rechteckförmig ausgebildet.

In der Gebrauchssituation werden die Windelseitenteile in Überlappung mit der Außenseite des Vorderbereichs des Hauptteils gebracht, damit die im Bereich der jeweiligen freien Enden an beiden Windelseitenteilen vorgesehenen Verschlussmittel auf die Außenseite des Hauptteils der Windel geschlossen werden können. Hierzu sind die Verschlussmittel und mindestens ein Bereich der Außenseite des Hauptteils als Verschlusssystem ausgebildet. Insbesondere weisen die Verschlussmittel hierzu mechanische Verschlusselemente wie Kletthakenelemente auf, insbesondere auch in Kombination mit haftklebenden Bereichen, vermittels derer die Verschlussmittel lösbar haftend mit der Außenseite des Hauptteils in Eingriff bringbar sind. Hierzu hat es sich als vorteilhaft erwiesen, wenn die Außenseite des Hauptteils zumindest bereichsweise, vorzugsweise vollständig durch einen entsprechend ausgebildeten Vliesstoff gebildet ist. Alternativ ist möglich, ein separates Klettflauschelement auf der Außenseite des Hauptteils im Vorderbereich vorzusehen, welche als Landezone für die Verschlussmittel der Seitenabschnitte dient.

Vor der herstellerseitigen Verpackung der Inkontinenzwegwerfwindeln wird vorzugsweise der Hauptteil herstellerseitig mitsamt den auf sich selbst gefalteten Windelseitenteilen um eine erste und eine zweite jeweils in der Längsrichtung verlaufende Hauptteil-Falzachse nach innen auf sich selbst gefaltet, derart dass die beidseitigen Windelseitenteile in Dickenrichtung, also orthogonal zu einer die Längsrichtung und die Querrichtung einschließenden Ebene, in wenigstens teilweiser Überlappung zueinander zu liegen kommen. Weiter vorzugsweise wird vor der herstellerseitigen Verpackung der Inkontinenzwegwerfwindeln der Hauptteil herstellerseitig mitsamt den auf sich selbst gefalteten Windelseitenteilen und vorzugsweise im Anschluss an die zuvor beschriebene Faltung um in der Längsrichtung verlaufende Hauptteil-Falzachsen zusätzlich um eine oder zwei in der Querrichtung verlaufende Hauptteil-Falzachsen nach innen auf sich selbst gefaltet.

In den Figuren zeigen:
Figur 1a und 1b eine Draufsicht auf eine nach dem erfindungsgemäßen Verfahren hergestellte Inkontinenzwegwerfwindel,
Figur 2 eine Draufsicht auf eine in der Maschinenrichtung geförderte Materialbahn zur Bildung von Windelseitenteilen,
Figur 3 eine Draufsicht auf eine in der Maschinenrichtung geförderte Materialbahn nach dem Falten der Materialbahn,
Figur 4 einen Schnitt durch die Materialbahn der Figur 3 entlang A-A,
Figur 5 einen Schnitt durch eine Variante der gefalteten Materialbahn,
Figur 6a und 6b eine Draufsicht auf eine in der Maschinenrichtung geförderte Materialbahn zur Bildung von elastifizierten Windelseitenteilen,
Figur 7 schematisch das Abtrennen von einnutzigen Windelseitenteilabschnitten von einer Materialbahn zur Bildung von Windelseitenteilen,
Figur 8 schematisch das Anfügen der einnutzigen Windelseitenteilabschnitte an eine Hauptteilbahn,
Figur 9 schematisch das Anfügen von Materialstreifen an eine Materialbahn nach einer Variante der Erfindung,
Figur 10 schematisch das Abtrennen von einnutzigen Windelseitenteilabschnitten von einer Materialstreifenbahn nach Figur 9,
Figur 11 schematisch das Anfügen der einnutzigen Windelseitenteilabschnitte an eine Hauptteilbahn und das Vereinzeln der Hauptteilbahn mit daran angefügten einnutzigen Windelseitenteilabschnitten zur Bildung von Inkontinenzwegwerfwindeln.

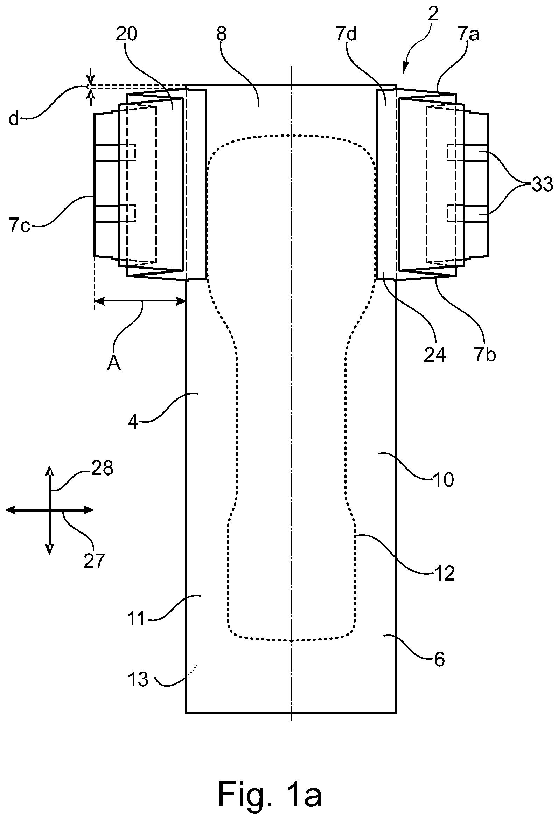

Figur 1a zeigt schematisch, nicht maßstabsgerecht eine Draufsicht auf die Innenseite, also die körperzugewandte Seite einer nach einem erfindungsgemäßen Verfahren hergestellte absorbierende Inkontinenzwegwerfwindel 2 des offenen Typs (nachfolgend auch kurz als "Windel" bezeichnet). Die Windel 2 umfasst einen Hauptteil 4 mit einem Vorderbereich 6, einem Rückenbereich 8 und einem in der Längsrichtung 28 dazwischen liegenden Schrittbereich 10. Außerdem angedeutet ist ein Absorptionskern 12, der üblicherweise zwischen chassisbildenden Materialien des Hauptteils 4, also insbesondere zwischen einem flüssigkeitsdurchlässigen, beispielsweise aus einem Vliesstoffmaterial gebildeten Topsheet 11 und einem im Wesentlichen flüssigkeitsundurchlässigen, beispielsweise aus einem Folienmaterial gebildeten oder ein Folienmaterial aufweisenden Backsheet 13 angeordnet ist. Das Backsheet 13 kann auch aus einem flüssigkeitsundurchlässigem Vliesstoff oder einem Vliesfolienlaminat ausgebildet sein, wobei die Vlieslage dann außen und die Folienlage innen zum Absorptionskern gerichtet zu liegen kommt. Seitlich neben den Längsrändern des Absorptionskerns 12 können elastische Elemente (nicht dargestellt) an den Windelhauptteil 4, insbesondere zwischen Topsheet 11 und Backsheet 12 angefügt sein. Die elastischen Elemente verlaufen im Wesentlichen in der Längsrichtung 28, also mit einer wesentlichen Komponente in Längsrichtung 28.

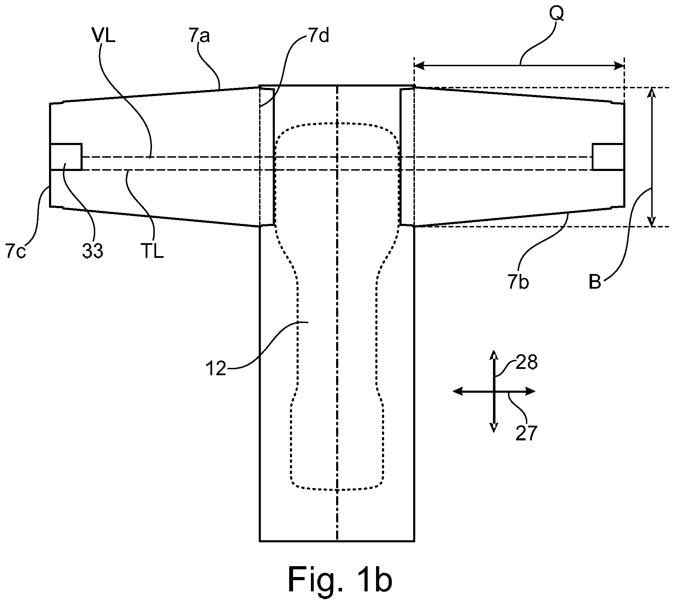

Die Windel 2 umfasst des Weiteren Windelseitenteile 20, die als zwei separate Vliesstoffkomponenten beidseits an den Hauptteil 4 angefügt sind. Die Windelseitenteile 20 weisen jeweils eine Faltanordnung auf. Die Faltanordnung wird in noch näher zu beschreibender Weise im Zuge der Herstellung der Windel eingebracht. Für die eingefaltete Konfiguration der Windelseitenteile 20 gilt vorzugsweise, dass eine Erstreckung A der auf sich selbst gefalteten Windelseitenteile 20 in der Querrichtung 27 über den jeweiligen hinteren seitlichen Längsrand hinaus und eine Erstreckung B (siehe Figur 1b) der auf sich selbst gefalteten Windelseitenteile in der Längsrichtung 28 derart bemessen sind, dass das Verhältnis der Erstreckungen A/B zueinander 0,25 < A/B <1,50, insbesondere 0,50 < A/B < 1,0 beträgt.

Die Windelseitenteile 20 sind in einem Überlappungsbereich 24 mit chassisbildenden Materialien des Hauptteils 4, also beispielsweise mit dem Backsheet 13 oder dem Topsheet 11 unlösbar verbunden. Die Windelseitenteile 20 sind dafür gedacht und bestimmt, im angelegten Zustand der Inkontinenzwegwerfwindel 2 mit dem Vorderbreich des Hauptteils 4 verbunden zu werden, um einen in Umfangsrichtung durchgehenden Hüftbereich der Windel 2 zu bilden. Dabei werden jeweils die Windelseitenteile 20 mit der Außenseite des Vorderbreichs des Hauptteils 4 in Überlappung gebracht. Hierzu sind an den hinteren Windelseitenteilen 20 Verschlussmittel 33, insbesondere mit mechanischen Verschlusshilfen wie Kletthaken, vorgesehen, welche auf der Außenseite des Vorderbereichs 6 des Hauptteils lösbar festlegbar sind. Die hinteren Windelseitenteile 20 sind vorzugsweise aus einem Vliesstoffmaterial, im dargestellten Fall aus einem PP-Spinnvlies gebildet. Das Flächengewicht des Vliesstoffmaterials der Windelseitenteile 20 beträgt 27 g/m². Die Faserstärke der das Vliesstoffmaterial bildenden Fasern beträgt zwei dtex. Ein jeweiliger äußerer Rand 7c der Windelseitenteile 20 ist in der Längsrichtung 28 kürzer ausgebildet als ein jeweiliger innerer Rand 7d der Windelseitenteile 20. Die Windelseitenteile 20 weisen außerdem einen oberen Rand 7a und einen unteren Rand 7b auf. Der untere Rand 7b ist auf der dem Schrittbereich 10 zugewandten Seite schräg zur Längsrichtung 28 der Windel 2 oder konturiert ausgebildet. Auch der obere Rand 7a ist schräg zur Längsrichtung 28 der Windel 2 oder konturiert ausgebildet. Der Abstand d der Windelseitenteile in der Längsrichtung zu dem hinteren Querrand des Hauptteils 4 beträgt vorliegend 5 mm.

Figur 1b zeigt ebenfalls nicht maßstabsgerecht eine Draufsicht auf die Innenseite, also die körperzugewandte Seite einer erfindungsgemäß hergestellten absorbierenden Inkontinenzwegwerfwindel des offenen Typs. Die Windelseitenteile sind in der vollständig entfalteten Konfiguration dargestellt. Im Unterschied zur der Ausführungsform nach Figur 1a weist jedes Windelseitenteil der Windel nach Figur 1b lediglich ein Verschlussmittel 33 auf. Die Erstreckung Q der eben ausgebreiteten Windelseitenteile in der Querrichtung beträgt 25 cm. Die Erstreckung B der Windelseitenteile in der Längsrichtung beträgt im Bereich der Anfügung an den Hauptteil 12 cm. Das Verhältnis Q/B beträgt mithin 2,08.

Erkennbar ist außerdem, dass eine in der Querrichtung erstreckte und das jeweilige Verschlussmittel auf der schrittzugewandten Seite gerade tangierende Linie TL den Saugkörper 12 schneidet. Auch eine die Windelseitenteile 20 in der Längsrichtung halbierende Linie VL, die in Querrichtung verläuft, schneidet den Saugkörper 12.

Der untere Rand 7b eines Windelseitenteils 20 ist auf der dem Schrittbereich 10 zugewandten Seite schräg zur Längsrichtung der Windel ausgebildet. Auch der obere Rand 7a ist schräg zur Längsrichtung der Windel ausgebildet. Mithin ist jeweiliger äußerer Rand 7c der Windelseitenteile in der Längsrichtung kürzer ausgebildet als ein jeweiliger innerer Rand 7d der Windelseitenteile.

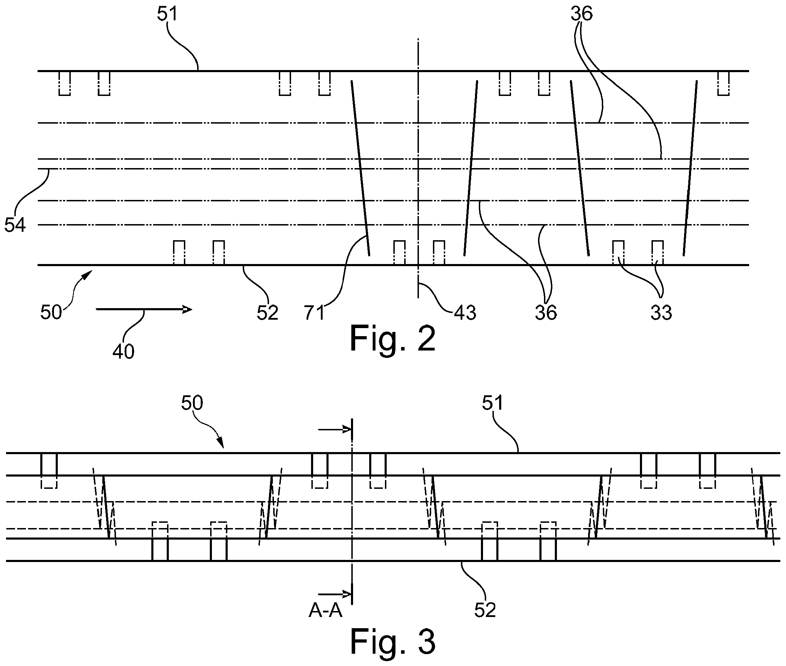

Die Figuren 2-7 zeigen schematisch das Zuführen und Konfigurieren von Materialbahnen 50, 50a, aus denen die Windelseitenteile 20 der Windel 2 gebildet werden. Figur 2 zeigt eine in der Maschinenrichtung 40 geförderte endlose Materialbahn 50. Die Materialbahn 50 weist einen ersten 51 und einen zweiten Materialbahnrand 52 auf. Schräg zu der Maschinenrichtung 40 sind an vorgelagerten Schnittstationen (nicht dargestellt) zur Bildung von im späteren Verlauf abzutrennenden einnutzigen Windelseitenteilabschnitten erste Schnitte ausgeführt, wobei erste Trennlinien 71 der ersten Schnitte sich nicht bis zu den Materialbahnrändern 51, 52 der Materialbahn 50 erstrecken, so dass die Bahn zunächst endlos bleibt. Die ersten Trennlinien 71 können wie dargestellt im einfachsten Fall als schräg zu der Maschinenrichtung 40 aber geradlinig verlaufende Schnitte ausgebildet sein. Die Trennlinien können vorteilhaft nach einer alternativen Ausführungsform konturiert beipielsweise S-förmig verlaufen. Nach einer weiteren Alternative können die ersten Trennlinien insbesondere Materialaussparungen bilden, derart dass jeweils eine lang gestreckte Öffnung in ovaler Form ausgebildet wird. Eine jeweilige Öffnung ist solchenfalls in der Ebene der Materialbahn 50 vollumfänglich von dem Bahnmaterial umschlossen.

In der Maschinenrichtung 40 aufeinanderfolgende erste Trennlinien 71 sind vorzugsweise jeweils symmetrisch in Bezug auf eine Spiegelachse 43 quer zur Maschinenrichtung 40 ausgerichtet.

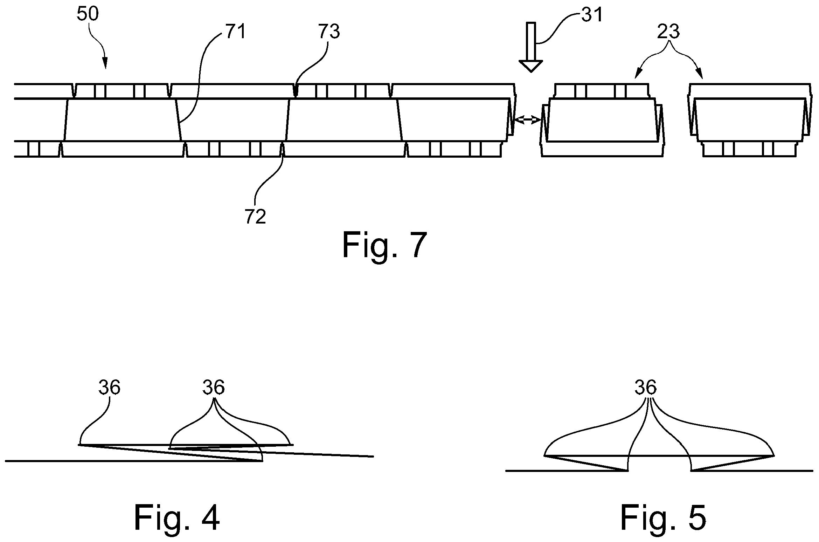

Vor oder nach dem Ausbilden der ersten Trennlinien 71 werden im beispielhaft dargestellten Fall je zwei Verschlussmittel 33 in der Fertigungsrichtung zwischen zwei aufeinanderfolgende erste Trennlinien 71 appliziert. Es handelt sich hierbei um an sich bekannte klebend oder mechanisch haftende Verschlussmittel 33, beispielsweise in Form von streifenförmigen Verschlusstapes. Angedeutet sind mit Bezugszeichen 36 spätere Falzlinien, welche beidseits einer Mittellängsachse 54 der Materialbahn 50 angeordnet sind. Figur 3 zeigt die Materialbahn 50 nach dem die Materialbahn an einer nicht dargestellten Falzstation um vier in Maschinenrichtung 40 verlaufende Falzlinien 36 zu einer Faltanordnung gefaltet ist, wobei alle vier Falzlinien 36 die ersten Trennlinien 71 kreuzen. Erkennbar ist, dass die Faltanordnung sich nicht über die Materialbahnränder 51, 52 hinauserstreckt. Figur 4 zeigt einen Querschnitt durch die gefaltete Materialbahn 50 der Figur 3 entlang A-A. Die Faltanordnung umfasst nach dieser Variante zwei Z-Falten, wobei die eine Z-Falte in die andere Z-Falte eingeschoben ist, also Teilabschnitte jeweiliger Z-Falten aufeinander zu liegen kommen. Figur 5 zeigt eine weitere Variante einer vorteilhaften Faltanordnung. Diese Faltanordnung umfasst zwei spiegelbildlich angeordnete Z-Falten.

Durch das Einfalten der Materialbahn 50 wird die Breite der geförderten Materialbahn von B1 = 34 cm auf B2 = 17 cm reduziert, also um den Faktor 0,5 reduziert.

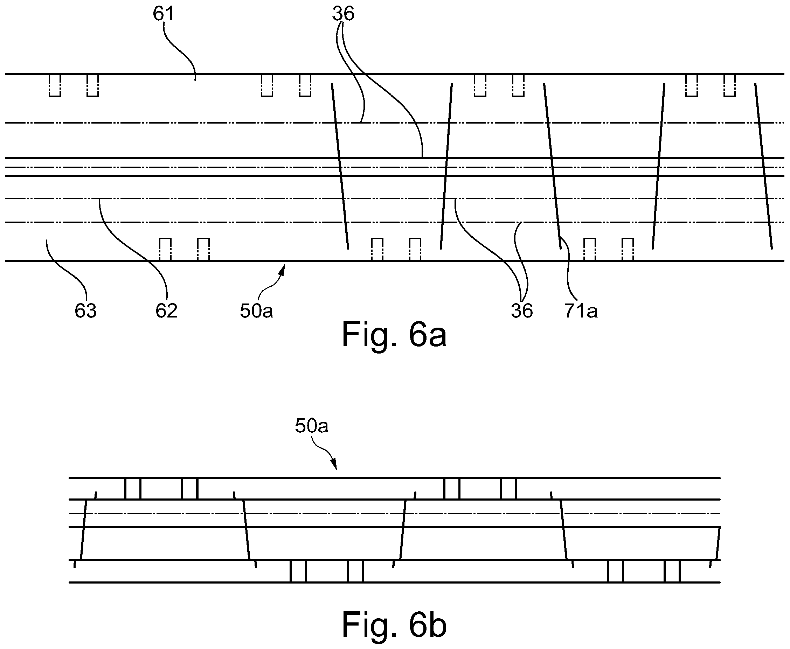

Während Figuren 2 und 3 eine Materialbahn 50 zur Erzeugung von im Wesentlichen unelastischen Windelseitenteilen 20 zeigen, ist in Figur 6a eine Materialbahn 50a dargestellt, welche zur Erzeugung von in Windelquerrichtung elastischen Windelseitenteilen bestimmt und geeignet ist.

Insbesondere kann die Materialbahn 50a ein von der Materialbahn 50 verschiedenes Material aufweisen. Das Material kann wie dargestellt quer zu der Maschinenrichtung 40 aus drei sich in Maschinenrichtung endlos erstreckenden Abschnitten bestehen. Ein erster unelastischer Abschnitt 61, ein an den ersten Abschnitt 61 angrenzender zweiter elastischer Abschnitt 62 und ein an den zweiten Abschnitt 62 angrenzender dritter unelastischer Abschnitt 63. Nach der dargestellten Variante verläuft einer der Faltenkanten 36 entlang des Materialübergangs zwischen dem ersten und zweiten Abschnitt. Erkennbar ist außerdem, dass die ersten Trennlinien 71 sich vollständig über die Quererstreckung des zweiten Abschnittes 62 hinwegerstrecken, das heißt die Materialübergänge zwischen erstem und zweitem Abschnitt und zweitem und drittem Abschnitt kreuzen.

Figur 6b zeigt die Materialbahn 50a nach dem die Materialbahn 50a an einer nicht dargestellten Falzstation um vier in Maschinenrichtung 40 verlaufende Falzlinien 36 zu einer Faltanordnung gefaltet ist, wobei alle vier Falzlinien 36 die ersten Trennlinien 71 kreuzen.

In einer nur schematisch angedeuteten Vereinzelungsstation 31 wird die in der Maschinenrichtung 40 immer noch endlose Materialbahn 50 (oder analog die Materialbahn 50a) in einnutzige Windelseitenteilabschnitte 23 getrennt (Figur 7). In der dargestellten Variante erfolgt dies durch zweite und dritte Trennlinien 72, 73, welche durch Schnitte gebildet werden, die ausgehend von einem jeweiligen Materialbahnrand 51, 52 sich U- oder V-förmig bis zu der ersten Trennlinie 71 erstrecken. Die Abtrennung kann an sich durch beliebige Trennverfahren insbesondere Schneiden, Stanzen oder Abreißen erfolgen. Auch könnten nach einer weiteren Variante Schwächungslinien eingebracht werden, die die endgültige Abtrennung der einnutzigen Windelseitenteilabschnitte durch Ausübung von Zug- oder Druckkräften ermöglichen.

Die Abtrennung der einnutzigen Windelseitenteilabschnitte von der Materialbahn 50a, mithin die Abtrennung von elastifizierten einnutzigen Windelseitenteilabschnitten für die Bildung von elastifizierten Windelseitenteilen erfolgt vorzugsweise analog wie zuvor für Materialbahn 50 beschrieben.

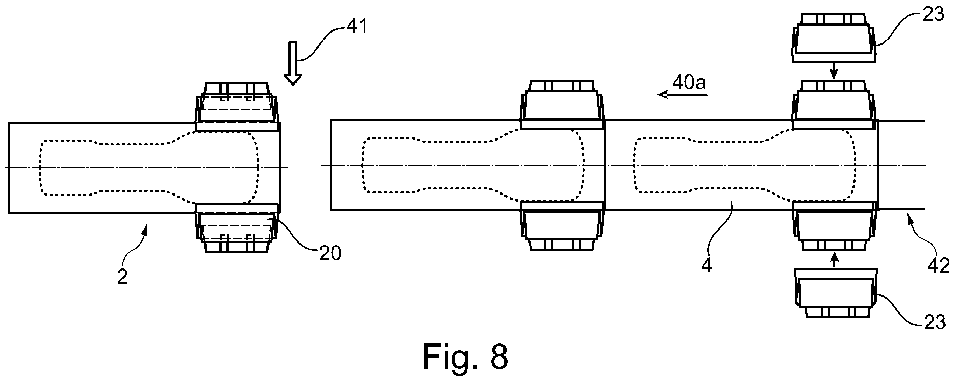

Die einnutzigen Windelseitenteilabschnitte 23 können dann mittels geeigneter Applikatoren (nicht dargestellt) auf eine Hauptteilbahn 42, welche entlang der Windelmaschinenrichtung 40a gefördert wird, appliziert und dort an Längsrandabschnitten des späteren Windelhauptteils 4 unlösbar befestigt werden (Figur 8), um Windelseitenteile 20 zu bilden.

Wenn vorstehend oder nachstehend von einer Hauptteilbahn die Rede ist, so kann sich dies auch auf eine Komponente einer den späteren Hauptteil bildenden endlosen Bahn, wie z.B. eine endlose Topsheetbahn oder eine endlose Backsheetbahn, beziehen, die dann nach der erfindungsgemässen Verfahrensführung mit weiteren den Hauptteil bildenden Komponenten, wie Saugkörperbahn, Topsheetbahn , Backsheetbahn, etc. verbunden wird. Unter einer Topsheetbahn wird die Bahn eines die Körperseite des Hauptteils bildenden Materials, wie eine Bahn eines in Gebrauch körperflüssigkeitendurchlassigen Materials, oder die Bahn eines hydrophoben Auslaufsperren (Cuffs) bildenden Vliesstoffmaterials verstanden.

Schließlich erfolgt an einer nur schematisch angedeuteten Windelvereinzelungsstation 41 die Vereinzelung der zuvor noch endlosen Hauptteilbahn 42 mit daran angefügten einnutzigen Windelseitenteilabschnitten quer zur Windelmaschinenrichtung 40a. (Figur 8). Die Vereinzelung erfolgt vorzugsweise derart, dass die Windelseitenteile 20 bildenden einnutzigen Windelseitenteilabschnitte mit einem Abstand d (siehe Figur 1a) von dem hinteren Querrand des Hauptteils angeordnet sind.

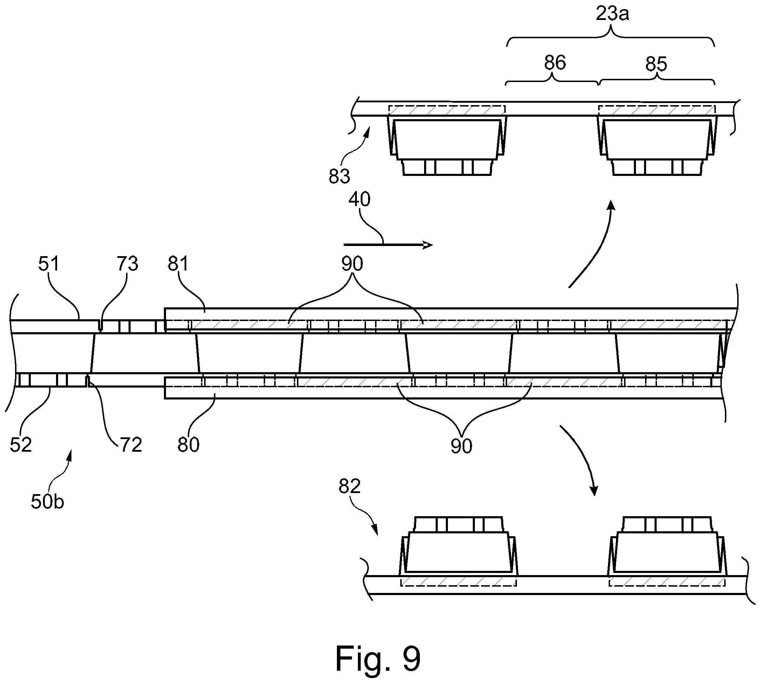

Figuren 9 bis 11 zeigen eine vorteilhafte Weiterbildung des erfindungsgemäßen Verfahrens. Hierbei werden nach der Ausbildung der zweiten und dritten Trennlinien 72, 73, aber noch vor der endgültigen Vereinzelung der Bahn in einnutzige Windelseitenteilabschnitte zunächst endlose Materialstreifen 80, 81 an den Materialbahnrändern 51, 52 angefügt. Die Anfügung erfolgt in schraffiert dargestellten Überlappungsbereichen (90), und zwar in der Maschinenrichtung 40 getaktet, so dass die Anfügung jeweils ausschließlich im Bereich der späteren inneren Ränder 71d der Windelseitenteile erfolgt, während die späteren äußeren Ränder 71c fügefrei verbleiben.

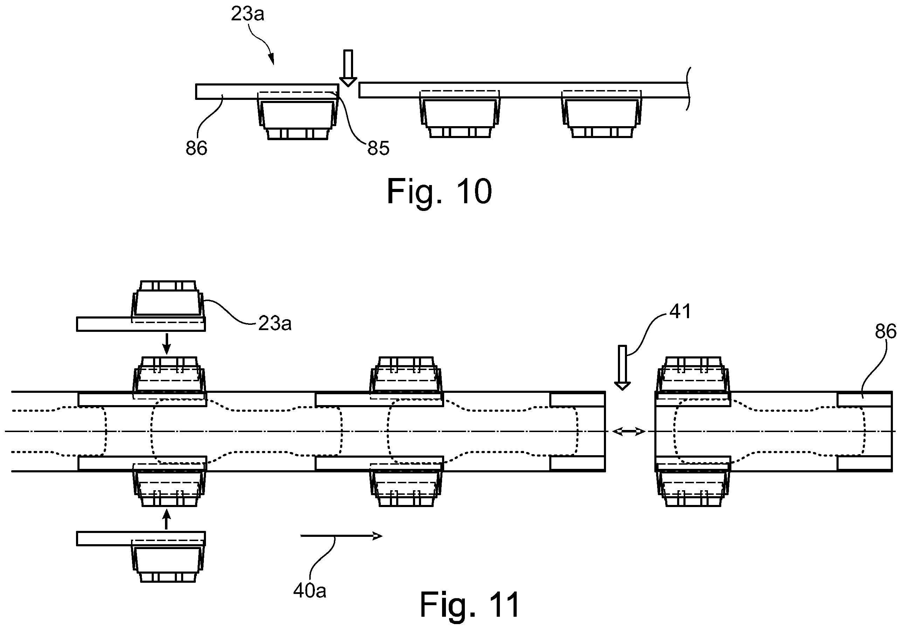

Nach dem Anfügen der Materialstreifen 80, 81 werden diese zur Ausbildung einer ersten endlosen Materialstreifenbahn 82 und einer zweiten endlosen Materialstreifenbahn 83 von einander wegbewegt, die Materialbahn 50b wird also in zwei Materialstreifenbahnen 82, 83 getrennt, wobei jeder der Materialstreifenbahnen 82, 83 eine in der Maschinenrichtung 40 getaktete Folge von einnutzigen Windelseitenteilabschnitten 23a aufweist, wobei ein jeweiliger einnutziger Windelseitenteilabschnitt 23a einen ein Windelseitenteil bildenden Abschnitt 85 und einen windelseitenteilfreien Abschnitt 86 aufweist. In einer nur schematisch angedeuteten Vereinzelungsstation werden die einnutzigen Windelseitenteilabschnitte 23a quer zur Maschinenrichtung 40 vereinzelt (Figur 10).

Diese einnutzigen Windelseitenteilabschnitte 23a können dann mittels geeigneter Applikatoren (nicht dargestellt) auf eine noch endlose Hauptteilbahn 42, welche entlang der Maschinenrichtung 40 gefördert wird, appliziert werden und dort an Längsrandabschnitten des späteren Windelhauptteils 4 unlösbar befestigt werden. Vorzugsweise erfolgt dies derart, dass der ein Windelseitenteil bildende Abschnitt 85 in dem Rückenbereich einer ersten Windel angefügt wird, während der windelseitenteilfreie Abschnitt 86 zumindest überwiegend in dem Vorderbereich einer zweiten der ersten Windel unmittelbar folgenden oder vorausgehenden zweiten Windel angefügt wird (Figur 11).

Schließlich erfolgt an einer nur schematisch angedeuteten Windelvereinzelungsstation 41 die Vereinzelung der zuvor noch endlosen Hauptteilbahn 42 mit daran angefügten einnutzigen Windelseitenteilabschnitten quer zur Windelmaschinenrichtung 40a. (Figur 11).

Nach dieser Variante kann der windelseitenteilfreie Abschnitt 86 beispielsweise der Verstärkung des Lagenverbundes unterhalb der Landezone des Vorderbereichs der Windel dienen. Der zweite windelseitenteilfreie Abschnitt verstärkt mithin zumindest bereichsweise den Materialaufbau der Zone, welche zumindest einen Teil des Auftreffbereichs für die Windelverschlüsse bildet.

Dem steht nicht entgegen, dass der Auftreffbereich für die Windelverschlüsse vorteilhaft auch durch die gesamte Außenseite des Vorderbereichs des Hauptteils der Windel gebildet sein kann.

Im Falle, dass der zweite windelseitenteilfreie Abschnitt auf der Außenseite der Windel angeordnet wird, kann der zweite windelseitenteilfreie Abschnitt 86 vorteilhaft selbst als Verschlussmaterial ausgebildet sein, insbesondere als ein Vliesmaterial, welches mit den Windelverschlüssen 33 in Eingriff bringbar ist.

Alternativ ließe sich im Falle, dass der zweite windelseitenteilfreie Abschnitt auf der Außenseite der Windel angeordnet wird, der zweite windelseitenteilfreie Abschnitt auch als ein Anti-Verschlussmaterial ausbilden, insbesondere als ein Vlies- oder Folienmaterial, welches mit den Windelverschlüssen gerade nicht in Eingriff bringbar ist. Damit kann der Nutzer falls gewünscht gezwungen werden, die Windelverschlüsse in einem zentralen Bereich der Außenseite des Vorderbereichs zu platzieren, um eine sichere und auf die Passform der Windel zugeschnittene Halteposition zu gewährleisten.

## Patentansprüche

1. Verfahren zum Herstellen von Inkontinenzwegwerfwindeln (2), mit einem Hauptteil (4), wobei der Hauptteil (4) einen Rückenbereich (8) umfasst, und mit beidseits an den Rückenbereich (8) angefügten Windelseitenteilen (20), wobei die Windelseitenteile (20) einen oberen Rand (7a) und einen unteren Rand (7b) aufweisen, wobei der obere Rand (7a) und der untere Rand (7b) schräg zur Längsrichtung (28) der Inkontinenzwegwerfwindel (2) oder konturiert verläuft und der untere Rand (7b) einen schräg zur Längsrichtung (28) der Inkontinenzwegwerfwindel (2) verlaufenden oder konturierten Beinöffnungsbereich begrenzt, umfassend die folgenden Schritte
- Fördern einer Materialbahn (50, 50a, 50b) in einer Maschinenrichtung (40), wobei die Materialbahn (50, 50a, 50b) einen ersten (51) und zweiten Materialbahnrand (52) aufweist
- Ausführen von ersten Schnitten durch die Materialbahn (50, 50a, 50b) hindurch und schräg zu der Maschinenrichtung (40) zur zumindest bereichsweisen Formung oberer Ränder (7a) und unterer Ränder (7b) einnutziger Windelseitenteilabschnitte (23, 23a), welche entlang der Materialbahn (50, 50a, 50b) in der Maschinenrichtung (40) alternierend angeordnet sind, derart, dass in der Maschinenrichtung (40) direkt aufeinanderfolgende einnutzige Windelseitenteilabschnitte (23, 23a) zueinander in der Ebene um 180° gedreht angeordnet sind, wobei erste Trennlinien (71) der ersten Schnitte sich nicht bis zu den ersten und zweiten Materialbahnrändern (51, 52) der Materialbahn (50, 50a, 50b) erstrecken, so dass die Materialbahn (50, 50a, 50b) zunächst endlos bleibt,
- Faltung der noch endlosen Materialbahn (50, 50a, 50b) wenigstens um zwei in Maschinenrichtung (40) verlaufende Falzlinien (36) zu einer Faltanordnung, wobei zumindest eine der Falzlinien (36) die ersten Trennlinien (71) kreuzt
- Vereinzelung der einnutzigen Windelseitenteilabschnitte (23, 23a) quer oder schräg zu der Maschinenrichtung (40).

2. Verfahren nach Anspruch 1, **dadurch gekennzeichnet, dass** die Faltanordnung eine Z-Falte umfasst.

3. Verfahren nach Anspruch 1 oder 2, **dadurch gekennzeichnet, dass** die Materialbahn (50, 50a, 50b) um wenigstens vier in Maschinenrichtung (40) verlaufenden Falzlinien (36) gefaltet ist.

4. Verfahren nach Anspruch 3, **dadurch gekennzeichnet, dass** die in Maschinenrichtung (40) verlaufenden Falzlinien (36) beidseits und insbesondere symmetrisch zu einer Mittellängsachse (54) der Materialbahn (50, 50a, 50b) angeordnet sind.

5. Verfahren nach Anspruch 4, **dadurch gekennzeichnet, dass** die Faltanordnung in Form einer Omega-Faltung ausgebildet ist.

6. Verfahren nach einem der vorgenannten Ansprüche, **dadurch gekennzeichnet, dass** bei der Vereinzelung der einnutzigen Windelseitenteilabschnitte (23) quer oder schräg zu der Maschinenrichtung (40) die einnutzigen Windelseitenteilabschnitte (23) direkt von der Materialbahn (50, 50a) abgetrennt werden.

7. Verfahren nach einem der vorgenannten Ansprüche 1-5, **dadurch gekennzeichnet, dass** vor der Vereinzelung der einnutzigen Windelseitenteilabschnitte (23a) quer oder schräg zu der Maschinenrichtung (40) endlose Materialstreifen (80, 81) an dem ersten und dem zweiten Materialbahnrand (51, 52) angefügt werden.

8. Verfahren nach Anspruch 7, **dadurch gekennzeichnet, dass** die Materialbahn (50b) in eine erste endlose Materialstreifenbahn (82) und eine zweiten endlose Materialstreifenbahn (83) getrennt wird, derart, dass jede der Materialstreifenbahnen (82, 83) eine in der Maschinenrichtung (40) getaktete Folge von einnutzigen Windelseitenteilabschnitten (23a) aufweist.

9. Verfahren nach einem der vorgenannten Ansprüche, **dadurch gekennzeichnet, dass** im Zuge der Vereinzelung der einnutzigen Windelseitenteilabschnitte (23, 23a) zweite und dritte Trennlinien (72, 73) insbesondere vermittels Schneiden oder Stanzen ausgehend von einem jeweiligen Materialbahnrand (51, 52) der Materialbahn (50, 50a) bis zu der ersten Trennlinie (71) ausgebildet werden.

10. Verfahren nach einem der vorgenannten Ansprüche, **dadurch gekennzeichnet, dass** die einnutzigen Windelseitenteilabschnitte (23, 23a) nach der Abtrennung von der Materialbahn (50, 50a, 50b) um wenigstens je eine weitere in Maschinenrichtung (40) verlaufende Falzlinie gefaltet werden.

11. Verfahren nach einem der vorgenannten Ansprüche, **dadurch gekennzeichnet, dass** die einnutzigen Windelseitenteilabschnitte (23, 23a) einer Hauptteilbahn (42) zugeführt werden und die Windelseitenteilabschnitte (23, 23a) zur Bildung von Windelseitenteilen (20) an der Hauptteilbahn (42) angefügt werden.

12. Verfahren nach einem der vorgenannten Ansprüche, **dadurch gekennzeichnet, dass** vor der Vereinzelung der einnutzigen Windelseitenteilabschnitte (23, 23a) Verschlussmittel (33) auf die Materialbahn (50, 50a, 50b) aufgebracht und dort festgelegt werden.

13. Verfahren nach einem der vorgenannten Ansprüche, **dadurch gekennzeichnet, dass** ein erster einnutziger Materialbahnabschnitt (23, 23a) ein linkes Windelseitenteil bildet und ein sich unmittelbar an den ersten einnutzigen Materialbahnabschnitt (23, 23a) anschließender zweiter einnutziger Materialbahnabschnitt (23, 23a) ein rechtes Windelseitenteil bildet.

14. Verfahren nach einem der vorgenannten Ansprüche 1-13, **dadurch gekennzeichnet, dass** die Inkontinenzwegwerfwindeln derart gefertigt werden, dass bei in einer Windelmaschinenrichtung (40a) aufeinander folgend geförderten Inkontinenzwegwerfwindeln der Rückenbereich (8) der einen Inkontinenzwegwerfwindel an den Vorderbereich (6) der anderen Inkontinenzwegwerfwindel anschließt.

15. Verfahren nach einem der vorgenannten Ansprüche, **dadurch gekennzeichnet, dass** die Materialbahn (50a) quer zu der Maschinenrichtung mindestens drei Abschnitte aufweist, umfassend einen ersten Abschnitt (61), ein an den ersten Abschnitt (61) angrenzender zweiter Abschnitt (62) und ein an den zweiten Abschnitt (62) angrenzender dritter Abschnitt (63).

16. Verfahren nach Anspruch 15, **dadurch gekennzeichnet, dass** der zweite Abschnitt (62) ein quer zu der Maschinenrichtung (40) elastisches Material umfasst oder daraus besteht.

17. Verfahren nach Anspruch 15-16, **dadurch gekennzeichnet, dass** der erste (61) und dritte Abschnitt (63) ein quer zu der Maschinenrichtung (40) im Wesentlichen unelastisches Material umfasst oder daraus besteht.

## Claims

1. Method for producing disposable incontinence diapers (2) having a main part (4), wherein the main part (4) comprises a back region (8), and having diaper side parts (20) joined to the back region (8) on both sides, wherein the diaper side parts (20) have an upper periphery (7a) and a lower periphery (7b), wherein the upper periphery (7a) and the lower periphery (7b) extend obliquely with respect to the longitudinal direction (28) of the disposable incontinence diaper (2) or in a contoured manner and the lower periphery (7b) delimits a leg opening region that extends obliquely with respect to the longitudinal direction (28) of the disposable incontinence diaper (2) or is contoured, the method comprising the following steps
- conveying a material web (50, 50a, 50b) in a machine direction (40), wherein the material web (50, 50a, 50b) has a first material-web periphery (51) and second material-web periphery (52),
- making first cuts through the material web (50, 50a, 50b) and obliquely with respect to the machine direction (40) so as to at least regionally form upper peripheries (7a) and lower peripheries (7b) of single-use diaper side-part portions (23, 23a) which are arranged alternately along the material web (50, 50a, 50b) in the machine direction (40), such that single-use diaper side-part portions (23, 23a) that follow one another directly in the machine direction (40) are arranged in a manner rotated through 180° in the plane with respect to one another, wherein first separating lines (71) of the first cuts do not extend as far as the first and second material web peripheries (51, 52) of the material web (50, 50a, 50b) and so the material web (50, 50a, 50b) initially remains endless,
- folding the still endless material web (50, 50a, 50b) at least about two folding lines (36), extending in the machine direction (40), to form a folded arrangement, wherein at least one of the folding lines (36) crosses the first separating lines (71),
- separating off the single-use diaper side-part portions (23, 23a) transversely or obliquely with respect to the machine direction (40).

2. Method according to Claim 1, **characterized in that** the folded arrangement comprises a Z-shaped fold.

3. Method according to Claim 1 or 2, **characterized in that** the material web (50, 50a, 50b) is folded about at least four folding lines (36) that extend in the machine direction (40).

4. Method according to Claim 3, **characterized in that** the folding lines (36) that extend in the machine direction (40) are arranged on both sides and in particular symmetrically with respect to a longitudinal centre axis (54) of the material web (50, 50a, 50b).

5. Method according to Claim 4, **characterized in that** the folded arrangement is in the form of an omega- shaped folded formation.

6. Method according to one of the preceding claims, **characterized in that**, when the single-use diaper side-part portions (23) are separated off transversely or obliquely with respect to the machine direction (40), the single-use diaper side-part portions (23) are detached directly from the material web (50, 50a).

7. Method according to one of the preceding Claims 1-5, **characterized in that**, before the single-use diaper side-part portions (23a) are separated off transversely or obliquely with respect to machine direction (40), endless material strips (80, 81) are joined to the first and the second material web periphery (51, 52).

8. Method according to Claim 7, **characterized in that** the material web (50b) is separated into a first endless material-strip web (82) and a second endless material-strip web (83) such that each of the material-strip webs (82, 83) has sequence, clocked in the machine direction (40), of single-use diaper side-part portions (23a).

9. Method according to one of the preceding claims, **characterized in that**, while the single-use diaper side-part portions (23, 23a) are being separated off, second and third separating lines (72, 73) are formed, in particular by means of cutting or punching, starting from a respective material-web periphery (51, 52) of the material web (50, 50a) as far as the first separating line (71).

10. Method according to one of the preceding claims, **characterized in that** after they have been separated from the material web (50, 50a, 50b), the single-use diaper side-part portions (23, 23a) are folded about at least one respective further folding line that extends in the machine direction (40).

11. Method according to one of the preceding claims, **characterized in that** the single-use diaper side-part portions (23, 23a) are fed to a main-part web (42) and the diaper side-part portions (23, 23a) are joined to the main-part web (42) to form diaper side parts (20).

12. Method according to one of the preceding claims, **characterized in that** before the single-use diaper side-part portions (23, 23a) are separated off, closure means (33) are applied to the material web (50, 50a, 50b) and secured there.

13. Method according to one of the preceding claims, **characterized in that** a first single-use material-web portion (23, 23a) forms a left diaper side part and a second single-use material-web portion (23, 23a) adjoining the first single-use material-web portion (23, 23a) forms a right diaper side part.

14. Method according to one of the preceding Claims 1-13, **characterized in that** the disposable incontinence diapers are manufactured such that, in the case of disposable incontinence diapers that are conveyed one after another in a diaper machine direction (40a), the back region (8) of one disposable incontinence diaper adjoins the front region (6) of the other disposable incontinence diaper.

15. Method according to one of the preceding claims, **characterized in that** the material web (50a) has, transversely with respect to the machine direction, at least three portions, comprising a first portion (61), a second portion (62) adjoining the first portion (61), and a third portion (63) adjoining the second portion (62).

16. Method according to Claim 15, **characterized in that** the second portion (62) comprises or consists of a material that is elastic transversely with respect to the machine direction (40).

17. Method according to Claims 15-16, **characterized in that** the first (61) and third portion (63) comprise or consist of a material that is substantially inelastic transversely with respect to the machine direction (40).

## Revendications

1. Procédé de fabrication de couches jetables pour incontinence (2), avec une partie principale (4), la partie principale (4) comprenant une zone dorsale (8), et avec des parties latérales de couche (20) jointes des deux côtés à la zone dorsale (8), les parties latérales de couche (20) présentant un bord supérieur (7a) et un bord inférieur (7b), le bord supérieur (7a) et le bord inférieur (7b) s'étendant obliquement par rapport à la direction longitudinale (28) de la couche jetable pour incontinence (2) ou de manière profilée, et le bord inférieur (7b) délimitant une zone d'ouverture de jambe s'étendant obliquement par rapport à la direction longitudinale (28) de la couche jetable pour incontinence (2) ou de manière profilée, comprenant les étapes suivantes :
- le transport d'une bande de matériau (50, 50a, 50b) dans une direction de la machine (40), la bande de matériau (50, 50a, 50b) présentant un premier (51) et un deuxième (52) bord de bande de matériau,
- la réalisation de premières coupes à travers la bande de matériau (50, 50a, 50b) et obliquement par rapport à la direction de la machine (40) pour former au moins par zones des bords supérieurs (7a) et des bords inférieurs (7b) de sections de partie latérale de couche à usage unique (23, 23a), qui sont agencés en alternance le long de la bande de matériau (50, 50a, 50b) dans la direction de la machine (40), de telle sorte que des sections de partie latérale de couche à usage unique (23, 23a) directement successives dans la direction de la machine (40) sont agencées en étant tournées de 180° les unes par rapport aux autres dans le plan, des premières lignes de séparation (71) des premières coupes ne s'étendant pas jusqu'aux premier et deuxième bords de bande de matériau (51, 52) de la bande de matériau (50, 50a, 50b), de telle sorte que la bande de matériau (50, 50a, 50b) reste tout d'abord sans fin,
- le pliage de la bande de matériau encore sans fin (50, 50a, 50b) au moins autour de deux lignes de pliage (36) s'étendant dans la direction de la machine (40) en un agencement de pliage, au moins l'une des lignes de pliage (36) croisant les premières lignes de séparation (71),
- l'individualisation des sections de partie latérale de couche à usage unique (23, 23a) transversalement ou obliquement par rapport à la direction de la machine (40) .

2. Procédé selon la revendication 1, **caractérisé en ce que** l'agencement de pliage comprend un pli en Z.

3. Procédé selon la revendication 1 ou 2, **caractérisé en ce que** la bande de matériau (50, 50a, 50b) est pliée autour d'au moins quatre lignes de pliage (36) s'étendant dans la direction de la machine (40).

4. Procédé selon la revendication 3, **caractérisé en ce que** les lignes de pliage (36) s'étendant dans la direction de la machine (40) sont agencées des deux côtés et notamment symétriquement par rapport à un axe longitudinal médian (54) de la bande de matériau (50, 50a, 50b).

5. Procédé selon la revendication 4, **caractérisé en ce que** l'agencement de pliage est réalisé sous la forme d'un pliage en oméga.

6. Procédé selon l'une quelconque des revendications précédentes, **caractérisé en ce que**, lors de l'individualisation des sections de partie latérale de couche à usage unique (23) transversalement ou obliquement par rapport à la direction de la machine (40), les sections de partie latérale de couche à usage unique (23) sont séparées directement de la bande de matériau (50, 50a).

7. Procédé selon l'une quelconque des revendications 1 à 5 précédentes, **caractérisé en ce qu'**avant l'individualisation des sections de partie latérale de couche à usage unique (23a) transversalement ou obliquement par rapport à la direction de la machine (40), des rubans de matériau sans fin (80, 81) sont joints au premier et au deuxième bord de bande de matériau (51, 52) .

8. Procédé selon la revendication 7, **caractérisé en ce que** la bande de matériau (50b) est séparée en une première bande de ruban de matériau sans fin (82) et une deuxième bande de ruban de matériau sans fin (83), de telle sorte que chacune des bandes de ruban de matériau (82, 83) présente une succession de sections de partie latérale de couche à usage unique (23a), cadencée dans la direction de la machine (40).

9. Procédé selon l'une quelconque des revendications précédentes, **caractérisé en ce qu'**au cours de l'individualisation des sections de partie latérale de couche à usage unique (23, 23a), des deuxièmes et troisièmes lignes de séparation (72, 73) sont formées, notamment au moyen d'un découpage ou d'un poinçonnage, en partant d'un bord de bande de matériau respectif (51, 52) de la bande de matériau (50, 50a) jusqu'à la première ligne de séparation (71).

10. Procédé selon l'une quelconque des revendications précédentes, **caractérisé en ce que** les sections de partie latérale de couche à usage unique (23, 23a) sont pliées après la séparation de la bande de matériau (50, 50a, 50b) autour d'au moins une autre ligne de pliage s'étendant dans la direction de la machine (40).

11. Procédé selon l'une quelconque des revendications précédentes, **caractérisé en ce que** les sections de partie latérale de couche à usage unique (23, 23a) sont amenées à une bande de partie principale (42) et les sections de partie latérale de couche (23, 23a) sont jointes à la bande de partie principale (42) pour former des parties latérales de couche (20).

12. Procédé selon l'une quelconque des revendications précédentes, **caractérisé en ce qu'**avant l'individualisation des sections de partie latérale de couche à usage unique (23, 23a), des moyens de fermeture (33) sont appliqués sur la bande de matériau (50, 50a, 50b) et y sont fixés.

13. Procédé selon l'une quelconque des revendications précédentes, **caractérisé en ce qu'**une première section de bande de matériau à usage unique (23, 23a) forme une partie latérale de couche gauche et une deuxième section de bande de matériau à usage unique (23, 23a) directement contigüe à la première section de bande de matériau à usage unique (23, 23a) forme une partie latérale de couche droite.

14. Procédé selon l'une quelconque des revendications 1 à 13 précédentes, **caractérisé en ce que** les couches jetables pour incontinence sont fabriquées de telle sorte que lorsque les couches jetables pour incontinence sont transportées successivement dans une direction de la machine à couches (40a), la zone dorsale (8) d'une couche jetable pour incontinence est contigüe à la zone avant (6) de l'autre couche jetable pour incontinence.

15. Procédé selon l'une quelconque des revendications précédentes, **caractérisé en ce que** la bande de matériau (50a) présente au moins trois sections transversalement à la direction de la machine, comprenant une première section (61), une deuxième section (62) adjacente à la première section (61) et une troisième section (63) adjacente à la deuxième section (62).

16. Procédé selon la revendication 15, **caractérisé en ce que** la deuxième section (62) comprend ou est constituée d'un matériau élastique transversalement à la direction de la machine (40).

17. Procédé selon les revendications 15 à 16, **caractérisé en ce que** la première (61) et la troisième (63) section comprennent ou sont constituées d'un matériau essentiellement inélastique transversalement à la direction de la machine (40).
